# EUROPEAN PATENT SPECIFICATION

(11) **EP 1 481 664 B1**
(45) Date of publication and mention of the grant of the patent: **22.12.2010**
(21) Application number: 04252187.2
(22) Date of filing: 14.04.2004
(51) Int. Cl.: A61K 8/81, A61K 8/35, A61Q 19/04

(54) **Stable cosmetic compositions comprising a self-tanning agent**
Stabile kosmetische Zubereitung enthaltend ein selbstbräunungsagens
Composition cosmétique stable contenant un agent autobronzant

(30) Priority: 27.05.2003 EP 03253316
(43) Date of publication of application: 01.12.2004
(73) Proprietor: THE PROCTER & GAMBLE COMPANY, Cincinnati, OH 45202 (US)
(72) Inventor: Stephens, Alison Fiona, Cookham Berkshire SL6 9LA (GB); Crook, Teresa Barbara, Camberley Surrey GU17 9LY (GB)
(74) Representative: Wilding, Richard Alan

(56) References cited:
- WO-A-88/01164
- WO-A-98/00098
- US-A- 5 232 688
- US-A- 5 302 378
- US-A1- 2001 051 686
- US-B1- 6 197 287
- US-B1- 6 231 837
- "Simulgel (TM) NS - An emulsifying/thinkening polymer ... for New Sensations" documentation by the manufacturer SEPPIC, Paris (France), published 02/July/2001 XP002260687
- T. Kurz: "the color of DHA tan" , venezia , pages 361-366

## Description

### TECHNICAL FIELD OF THE INVENTION

The present invention is directed towards the use of cosmetic compositions comprising a self-tanning agent having good shelf stability, good application characteristics and imparting excellent skin colouration without unacceptable orange discoloration as defined in the claims.

### BACKGROUND OF THE INVENTION

A wide variety of cosmetic compositions containing self-tanning agents have been used to increase the pigmentation of the skin. These compositions have been used to create artificial tans, bronzing the skin in a similar fashion to exposure to the sun. These compositions are typically quite unstable, having poor shelf life. This is thought to be due to the highly reactive nature of the self-tanning agents therein.

The prior art teaches several solutions to the problem of stability of self-tanning agents in cosmetic compositions. US 6,231,837 B1 teaches the use of polyethoxyglycols such as ethoxydiglycol and polyols to stabilise self-tanning agent-containing compositions. WO 98/00098 teaches the combination of self-tanning agents and cross-linked non-emulsifying siloxane elastomers to form stable emulsions. US 6,197,287 discloses anionic polymers and compositions comprising them, including self-tanning agents. However, these solutions are not entirely satisfactory at maintaining the stability of self-tanning agent-containing formulations or have other disadvantages such as poor skin feel when topically applied.

It is desirable to provide stable cosmetic compositions comprising a self-tanning agent to provide a unique level of skin tanning that lifts the skin tone. Furthermore, it is desirable to provide a cosmetic composition comprising both self-tanning skin colouring agents and chronic skin care actives that act synergistically to maximise skin tone enhancement. It is further desirable to include chronic skin regulating agents in the compositions of the present invention to improve the skin barrier and so minimise any areas of dry or compromised skin. This is desirable as dry skin areas have uneven uptake of colour resulting in a blotchy or patchy result.

A supplier brochure by SEPPIC (XP-002260687), entitled "Simulgel ™ NS - An emulsifying/thickening polymer ... for New Sensations" disclose a self-tan gel-cream composition comprising 5% by weight Simulgel NS and 2.00% by weight dehydroxyacetone, but no humectant.

A summary of a poster by T. Kurz (P043, "The Color of DHA Tan") presented at an International Federation of Societies of Cosmetic Chemists (IFSCC) conference in Venezia (1994) relates to the possibilities to influence the hue of a DHA tan. The influence of having a moisturizer is discussed, and according to this document, "addition of 10% moisturizer does not increase the intensity of the tanning, it even seems to lead to a slight reduction of it". Different results were obtained at a level of 20% moisturizer.

WO98/00098 relates to self-tanner compositions comprising a different thickening system (a crosslinked non-emulsifying siloxane elastomer) than the claimed invention. WO98/00098 recognizes that "polyhydric alcohols enhance skin colorization of the self-tanning agent", but however recommends that the level of polyhydric alcohol humectant should be very high, "optimally" of from 25% to 35%.

US6,231,837B1 relates to a self-tanner composition with improved stability of the self-tanning agent and does not discuss the problem of improving the intensity of skin coloration. US5,232,688 discloses self-tanning compositions optionally comprising very high level of propylene glycol to improve the intensity of the color of the skin. The minimum level of propylene glycol recommended is 15%, while the optimal levels are between 25% and 45% by weight. US5,302,378 discloses self-tanning cosmetic compositions, and recommends level of propylene glycol of "optimally" between 25% and 45%.

### SUMMARY OF THE INVENTION

The present invention is directed to uses of cosmetic compositions according to claim 1.

The present invention is further directed towards providing skin care kits, methods of regulating skin condition and methods of manufacture of the cosmetic composition.

The compositions of the invention are stable, useful for topical application and for providing essentially immediate (i.e. acute) improvement in skin appearance following topical application. Without being limited by theory, it is believed that this acute improvement results at least in part from an immediate increase in skin tone by the self-tanning skin colouring agent.

The compositions of the present invention have very good acute benefits on application, namely producing good skin tone without the skin appearing unnatural or orange in effect.

These and other features, aspects, and advantages of the present invention will become evident to those skilled in the art from a reading of the present disclosure.

### DETAILED DESCRIPTION OF THE INVENTION

All weights, measurements and concentrations herein are measured at 25°C on the composition in its entirety, unless otherwise specified.

Unless otherwise indicated, all percentages of compositions referred to herein are weight percentages and all ratios are weight ratios.

Unless otherwise indicated, all molecular weights are weight average molecular weights. Unless otherwise indicated, the content of all literature sources referred to within this text are incorporated herein in full by reference.

Except where specific examples of actual measured values are presented, numerical values referred to herein should be considered to be qualified by the word "about".

The cosmetic compositions of the present invention comprise a self-tanning agent. As used herein, the term "self-tanning agent" includes α-hydroxy aldehydes and ketones such as dihydroxyacetone and structurally related compounds. This definition includes all such agents that are similarly useful in producing or inducing the artificial tanning process in human skin. Accordingly, the compositions of the present invention comprise an α-hydroxy aldehyde or ketone of the formula (I): 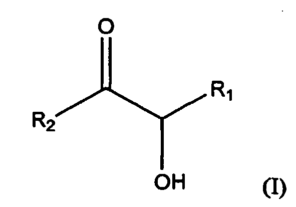 wherein R₁ is H, CH₂OH, CHOHCH₂OH, CH(OH)CH(=O), CH(OCH₃)CH(=O), CH(NH₂)CH(=O), or CH(NH-Phenyl)CH(=O); and R₂ is H or CH₂OH. Dihydroxyacetone (DHA) itself may be represented by the following general structural formula:

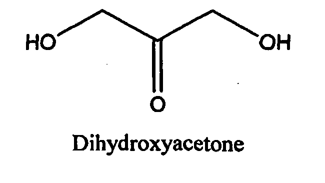

A number of other compounds are already known in the art as capable of producing or inducing the same artificial tanning process in human skin as is produced or induced by DHA. Some of these are structurally similar to DHA, and include the following:

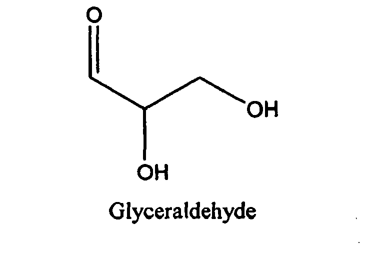

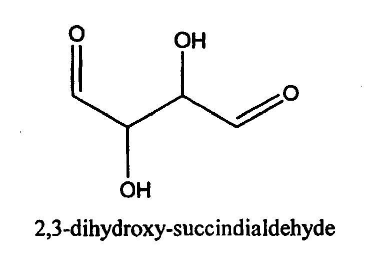

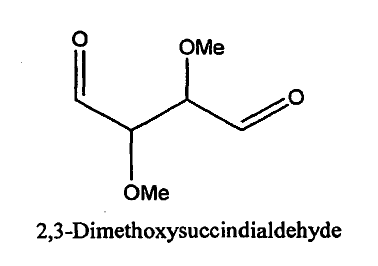

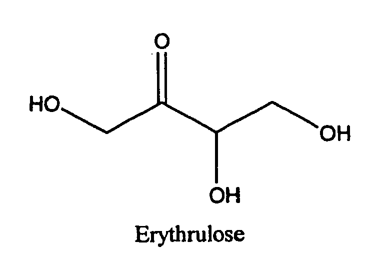

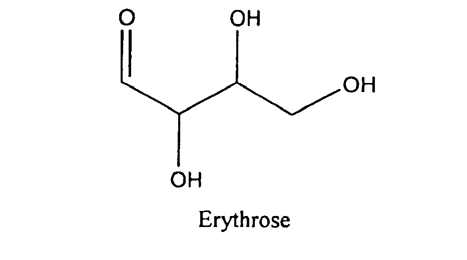

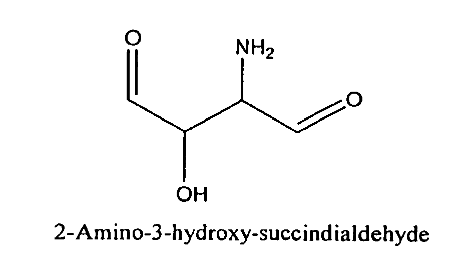

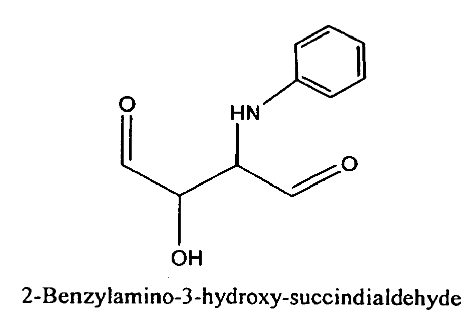

Preferably, the self-tanning agent comprises DHA, erythrulose, or mixtures thereof, more preferably DHA. Preferably the compositions of the present invention comprise from 0.1% to 10% of the self-tanning agent preferably less than 5%. More preferable, the compositions of the present invention comprise from 1.0% to 3.5%, more preferably still from 1.2% to 2.5% of the self tanning agent.

The cosmetic compositions of the present invention also comprise a cross-linked anionic polyelectrolyte polymer. As used herein, " cross-linked anionic polyelectrolyte polymer" includes non-linear polymers in the form of a three-dimensional network that is insoluble in water but swellable in water and thus leading to the production of a chemical gel. The composition according to the invention comprises cross-linked units. The cross-linked anionic polyelectrolyte polymer comprises at least one monomer possessing a strongly acidic function, co-polymerized with at least one hydroxy alkyl acrylate neutral monomer.

Preferably, the compositions of the present invention comprise a cross-linked anionic polyelectrolyte polymer that is the result of the copolymerization of its precursor monomers that is carried out at pH 4.0 or below. Preferably the anionic polyelectrolyte comprises from 58.7% to 89.9999% by weight of the polyelectrolyte polymer of a monomer having a strongly acidic function. As used herein, the term "monomer having a strongly acidic function" includes monomers having a pKa of less than 3. The pKa is measured by titration of the monomer having a strongly acidic function with a strong base in aqueous solution according to methods well known in the art. The strongly acidic function of the monomer containing it is preferably a sulphonic acid function or a phosphonic acid function, partially or totally salified. Non-limiting examples of monomers having a strongly acidic function group suitable for use herein include partially or totally salified 2-methyl-2-[(1-oxo-2-propenyl)amino]-1-propanesulphonic acid, partially or totally salified styrenesulfonic acid, or mixtures thereof. Preferably, the monomer comprises the partially or totally salified alkali metal salt or an ammonium salt of 2-methyl-2-[(1-oxo-2-propenyl)amino]-1-propanesulphonic acid.

Non-limiting examples of the at least one hydroxy alkyl acrylate neutral monomer suitable for use herein include 2-hydroxy-ethyl acrylate, 2,3-dihydroxypropyl acrylate, 2-hydroxy-ethyl methacrylate and 2,3-dihydroxypropyl methacrylate, or an ethoxylated derivative, with a molecular weight from 400 to 1000 of each of these esters, or mixtures thereof, preferably 2-hydroxy-ethyl acrylate. Polyelectrolyte polymers useful herein preferably comprise from 9.9999% to 40% by weight of the polyelectrolyte polymer of the at least one hydroxy alkyl acrylate neutral monomer.

More preferably, the compositions of the present invention comprise a reverse latex polymer comprising a branched and/or cross-linked anionic polyelectrolyte polymer, said polymer comprising partially or totally salified 2-methyl-2-[(1-oxo-2-propenyl)amino]-1-propanesulfonic acid, copolymerized with 2-hydroxyethyl acrylate.

The polymers for use in the present invention preferably comprise an anionic polyelectrolyte that is crosslinked with a diethylenic or polyethylenic compound at levels of from 0.0002% to 1.3%, preferably from 0.001% to 0.8%, more preferably from 0.01% to 0.6% by weight of the polyelectrolyte polymer. Non-limiting examples of crosslinking agents agents suitable for use herein include ethylene glycol dimethacrylate, sodium diallyloxyacetate, ethylene glycol diacrylate, diallylurea, trimethylolpropane triacrylate, methylene bisacrylamide, or mixtures thereof, preferably methylene bisacrylamide.

Preferably, the cosmetic compositions of the present invention comprise from 0.1% to 2.0% of the cross-linked anionic polyelectrolyte polymer, more preferably from 0.2% to 1.2%, more preferably still from 0.4% to 0.8%.

Non-limiting examples of commercially available cross-linked anionic polyelectrolyte polymer compositions suitable for use herein include Simulgel NS™, available from Societe D'Exploitation de Produits Pour Les Industries Chimiques (SEPPIC), Paris, France.

The compositions of the invention comprise a polyhydric alcohol humectant being glycerine, at a level of 5 to 13% by weight.

It has been found that glycerine is desirable to counteract the drying effect that self-tanning agents have when topically applied, and also to chronically hydrate the skin in order to improve its skin barrier function. It has also been surprisingly found that glycerine increases the coloration of skin by the self tanning agent claimed (e.g. DHA), even when present at level lower than 20%. The improved coloration effect can be shown by measuring the skin coloration (e.g. on the forearm) obtained with a product according to the invention versus the same product without a polyhydric alcohol humectant. Skin coloration measurements can be made according to conventional means, for example using a Minolta™ Chromameter CR-300.

This increased coloration effect was particularly noticeable at levels of polyhydric alcohol humectant from 5 to 13% by weight, preferably from 8 to 13% and for self-tanning agent levels below levels used in conventional marketed self tanning products, i.e. levels at or below 5%, preferably at or below 4%, more preferably at or below 3%. Using lower levels of skin tanning agent also benefits the skin as these tend to damage skin over repeated uses. Whilst not wishing to be bound by theory, Applicant believes that the glycerine increases the water content of the skin horny layer, which in turn favours the self tanning process. It is also believed that glycerine may improve the skin condition, minimising any areas of dry or compromised skin. This is desirable as dry skin areas have uneven uptake of colour resulting in a blotchy or patchy result.

The compositions of the present invention further have a pH of from 3.5 to 4.5, preferably from 3.7 to 4.2. It has surprisingly been found that the polymers as defined in the present invention are stable at these low pHs, and in the presence of self-tanning agents. It has also surprisingly been found that by combining the polymers of the present invention with self-tanning agents at low pHs, stable emulsions can be formed that deliver relatively high proportions of active self-tanning agent to the skin following topical application, without the discoloration of the composition often associated with compositions comprising self-tanning agents. Without wishing to be bound by theory, it is believed that the combination of the polymers herein, and the low pH of the composition prevent the formation of methyl glyoxal, a degradation product of DHA. It has been suggested in US 6,231,837 B1 that methyl glyoxal is responsible for the development of discoloration in formulations comprising DHA, and is also responsible for the production of an undesirable orange pigmentation when DHA is applied to the skin and is not present in a stabilised form. This may be the reason why the compositions of the present invention deliver self-tanning agents to the skin in a form that provides a natural tanning appearance, without the associated orange discoloration often associated with compositions containing self-tanning agents.

The compositions of the present invention may additionally comprise other humectants, including sodium 2-pyrrolidone-5-carboxylate (NaPCA); guanidine; glycolic acid and glycolate salts (e.g. ammonium and quaternary alkyl ammonium); aloe vera in any of its variety of forms (e.g., aloe vera gel); hyaluronic acid, precursors and derivatives thereof (e.g., glucosamine and salt derivatives such as sodium hyaluronate); lactamide monoethanolamine; acetamide monoethanolamine; urea; and mixtures thereof.

The compositions of the present invention may further comprise from 0.1% to 15% of a skin care active that is stable in self-tanning systems. Preferably, the compositions of the present invention comprise from 0.25% to 5% of a skin care active. Stable skin care actives useful herein include betaine and its derivatives, tocopherol esters, skin lipids, or mixtures thereof. Incorporation of skin care actives in the present compositions enhances the skin benefit properties by providing reduced water loss and/or providing a desquamatory, keratolytic and rejuvenating effect when topically applied.

One class of skin care actives suitable for use herein are the betaines. Betaines suitable for use herein include those with a molecular weight of from greater than 75 to less than 260, preferably from greater than 75 to less than 200 and more preferably from greater than 75 to less than 180.

Betaines useful in the present invention comprise quaternary ammonium salts represented by the general formula (II) below: 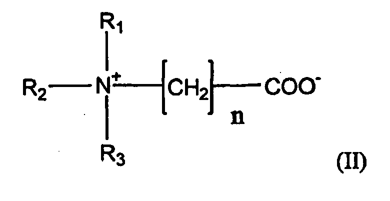 wherein R₁, R₂, and R₃ are independently selected from -H, -CH₃, -CH₂CH₃, and [-CH₂CH(OH)R₄, wherein R₄ is selected from -H, and C1 to C4 alkanes]; and wherein n = an integer from 1 to 3, preferably 1. Preferably R₁, R₂, and R₃ are all -CH₃, and n is 1; or R₁, R₂, and R₃ are all -CH₃, and n is 2; or R₁ and R₂ are -CH₃, R₃ is -H, and n is 1; or R₁ is -CH₃, R₂ and R₃ are -H, and n is 1; or R₁ and R₂ are [-CH₂CH(OH)R₄, R₄ is -H or -CH₃], R₃ is -H, and n is 1.

Further examples of betaines and derivatives thereof suitable for use in the present invention include betaines conforming to formula (II), wherein any two of R₁, R₂, and R₃ are independently selected from -H, and -CH₃, and the third moiety of R₁, R₂, and R₃ is selected from-(CH₂)mCH₃ wherein m is 4 or 5; and wherein n = an integer from 1 to 3, preferably 1; preferably wherein R₁ and R₂ are -CH₃, R₃ is -(CH₂)mCH₃ wherein m is 4, and n is 1.

Another class of betaine derivatives suitable for use in the present invention include cholines conforming to the general formula (III): 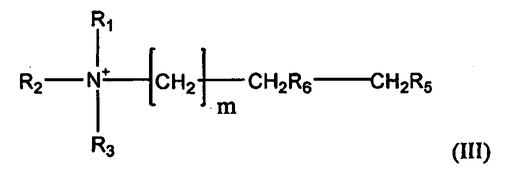 wherein R₁, R₂, R₃ R₄ are defined as in part (i) above; and wherein m is an integer from 0 to 2, and R₅ is selected from PO₄, SO₄ and SO₃, and R₆ is selected from H or OH. Preferred are cholines wherein R₁, R₂, and R₃ are -CH₃; m is 0; R₆ is H and R₅ is PO₄; or taurines wherein R₁, R₂, and R₃ are -CH₃; m is 0; and R₅ is SO₃, R₆ is H, or R₁, R₂, and R₃ are -CH₃; m is 1; R₆ is OH and R₅ is SO_{3.}

Further betaine derivatives suitable for use in the present invention are those comprising proline, carnitine, trimethylamineoxide, tricine, dimethyl proline and mixtures thereof.

Preferred for use herein are betaine or derivatives thereof comprising trimethylglycine, dimethyl glycine, sarcosine, trimethyl alanine, tricine, dimethyl proline, bicine, gamma-butyro betaine, trimethylamineoxide, proline, carnitine, and mixtures thereof, more preferably comprising trimethylglycine, tricine and dimethyl glycine; and mixtures thereof.

Non-limiting examples of preferred betaines or derivatives for use herein are identified immediately above. Non-limiting examples of preferred betaines or derivatives include trimethylglycine hydrate, available as TEGOCARE AP (RTM), from T.H. Goldschmidt (Germany), proline, available as proline, from Huls-Degussa (Germany), bicine, available as bicine, from Sigma Chemical (USA), and dimethylglycine, available as dimethylglycine from Sigma Chemical (USA).

Another class of skin care actives suitable for use herein includes the tocopherol esters, materials formed as a result of the condensation reaction between tocopherol and carboxylic acids. As used herein, "tocopherol esters" includes those materials conforming to the general formula (IV): 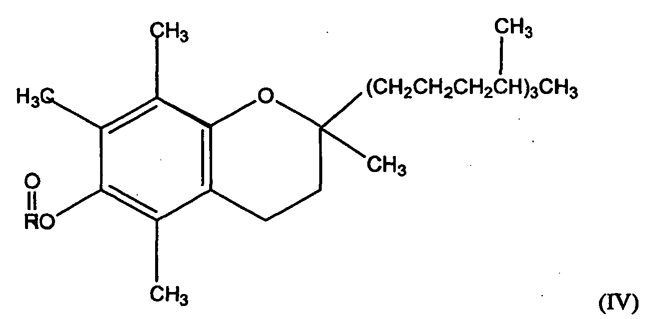 wherein, R=OO is selected from linear, branched and cyclic carboxylic acids, preferably acetic acid, linoleic acid, nicotinic acid, oleic acid, or mixtures thereof. More preferably, the skin care active comprises tocopherol acetate, tocopherol nicotinate, or mixtures thereof. Non-limiting examples of commercially available tocopherol esters include tocopherol nicotinate from Ennagram UK Ltd,, London, UK

A further class of skin care actives suitable for use in the present invention are the skin lipids. Skin lipids perform an integral function of maintaining the water holding ability of the stratum corneum, regulating skin health. Non-limiting examples of skin lipids suitable for use herein include ceramides, cholesterols, fatty acids, or mixtures thereof. Ceramides, the primary component of skin lipids, and are comprised of sphingolipids consisting of a long chain amino alcohol (sphingosine or one of it's derivatives) to which a long chain fatty acid is linked via an amide bond. Natural or synthetic sources can be used, as can pseudo-ceramides. Preferably, the skin care active herein comprises ceramide, cholesterol, sphingosine, or mixtures thereof. Non-limiting examples of commercially available blends of skin lipids suitable for use herein include SK Influx from Cosmoferm.

The compositions of the present invention may further comprise a natural gum thickener. It has been found that the rheology and stability of the present compositions can be suitably modified and improved by the addition of low levels of natural gum thickeners, without the compositions becoming too stringy, or tacky. Natural gum thickeners suitable for use herein include xanthan gum, guar gum or its derivatives, chitosan, alginates, carragenan, locust bean gum, sclerotium, pectin, starches or their derivatives, or mixtures thereof, preferably xanthan gum. Where present, the natural gum thickeners are preferably present at levels of from 0.05% to 3%, more preferably from 0.05% to 1%, more preferably still from 0.05% to 0.5%.

The compositions of the invention are preferably formulated so as to have a product viscosity of at least 4,000 mPa.s and preferably in the range from 4,000 to 300,000 mPa.s, more preferably from 8,000 to 250,000 mPa.s and especially from 10,000 to 200,000 mPa.s and even more especially from 20,000 to 100,000 mPa.s (25°C, neat, Brookfield RVT, T-C Spindle at 5 rpms and Heliopath Stand). The compositions of the present invention may be formulated as an emulsion.

The cosmetic compositions herein are in the form of a oil-in-water emulsion. Preferably the cosmetic compositions herein are oil-in-water emulsions wherein the composition comprises one or more oil phases in an aqueous continuous phase, each oil phase comprising a single oily component or a mixture of oily components in miscible or homogeneous form. Different oil phases contain different materials, or different combinations of materials, from each other. The total level of oil phase components in the compositions of the invention is from 1% to 60%, preferably from 1% to 30%, more preferably from 3% to 20% and most preferably from 5% to 15%.

In preferred embodiments, the oil phase preferably comprises oily components such as a natural or synthetic oils selected from mineral, vegetable, and animal oils, fats and waxes, fatty acid esters, fatty alcohols, fatty acids and mixtures thereof. Preferred for use herein are for example, saturated and unsaturated fatty alcohols such as behenyl alcohol, cetyl alcohol and stearyl alcohol and hydrocarbons such as mineral oils or petrolatum.

The present compositions may further comprise a silicone phase. The silicone phase can comprise one or more silicone components such as silicone fluids, gums, and mixtures thereof. The, or each, silicone phase generally comprises from 0.1 % to 20%, preferably from 0.2% to 10%, more preferably from 0.3% to 5%, of the composition.

Silicone components can be fluids, including straight chain, branched and cyclic silicones. Suitable silicone fluids useful herein include silicones inclusive of polyalkyl siloxane fluids, polyaryl siloxane fluids, cyclic and linear polyalkylsiloxanes, polyalkoxylated silicones, amino and quaternary ammonium modified silicones, polyalkylaryl siloxanes or a polyether siloxane copolymer and mixtures thereof. The silicone fluids can be volatile or non-volatile.

The silicone components can also comprise silicone gums. The term "silicone gum" herein includes high molecular weight silicones having a weight average molecular weight in excess of about 200,000 and preferably from about 200,000 to about 4,000,000. Included are non-volatile polyalkyl and polyaryl siloxane gums. In preferred embodiments, a silicone oil phase comprises a silicone gum or a mixture of silicones including the silicone gum.

Useful herein are silicone/gum fluid blends. Preferred silicone-gum fluid blend based component for use in the compositions herein is a dimethiconol gum having a molecular weight of from 200,000 to 4,000,000 along with a silicone fluid carrier with a viscosity of 0.65 to 100 mm².s⁻¹. An example of this silicone component is Dow Coming Q2-1503 (85% 5 mm².s⁻¹ Dimethicone Fluid/15% Dimethiconol) and Dow Coming Q2-1501 available from Dow Corning.

The topical compositions of the present invention preferably comprise emollient materials including branched chain hydrocarbons having an weight average molecular weight of from 100 to 15,000, preferably from 100 to 1000; compounds of formula V: 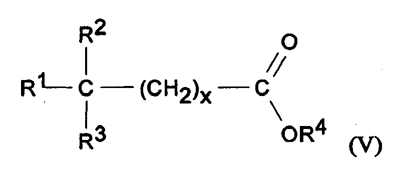 wherein R¹ is selected from H or CH₃, R², R³ and R⁴ are independently selected from C₁-C₂₀ straight chain or branched chain alkyl, and x is an integer of from 1-20; and compounds having the formula VI: 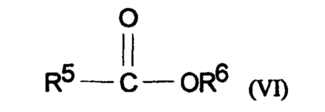 wherein R⁵ is selected from optionally hydroxy or C₁-C₄ alkyl substituted benzyl and R₆ is selected from C₁-C₂₀ branched or straight chain alkyl; and mixtures thereof.

Suitable branched chain hydrocarbons for use herein include isododecane, isohexadecane, isoeicosane, isooctahexacontane, isohexapentacontahectane, isopentacontaoctactane, and mixture thereof. Suitable ester emollient materials of Formula (VI) include but are not limited to C12-15 alkyl benzoates.

Preferred emollients for use herein are isohexadecane, isononyl isononanoate, methyl isostearate, isopropyl isostearate, and mixtures thereof. A further emollient suitable for use in the composition of the present invention is petrolatum.

The emollient material is preferably present in the compositions at a level of from about 0.1 % to about 10%.

The present compositions herein may comprise an emulsifier and/or surfactant, generally to help disperse and suspend the discontinuous phase within the continuous phase. For convenience hereinafter emulsifiers will be referred to under the term 'surfactants', thus 'surfactant(s)' will be used to refer to surface active agents whether used as emulsifiers or for other surfactant purposes such as skin cleansing. Known or conventional surfactants can be used in the composition, provided that the selected agent is chemically and physically compatible with essential components of the composition, and provides the desired characteristics.

The compositions of the present invention preferably comprise from 0.05% to 15% of a surfactant or mixture of surfactants. The exact surfactant or surfactant mixture chosen will depend upon the pH of the composition and the other components present. Surfactants suitable for use herein include non-ionic, cationic, anionic, zwitterionic, amphoteric surfactants, or mixtures thereof.

Preferred surfactants are nonionic. Among the nonionic surfactants that are useful herein are those that can be broadly defined as condensation products of long chain alcohols, e.g. C₈₋₃₀ alcohols, with sugar or starch polymers, i.e., glycosides. Preferred examples include a mixture of cetearyl glucosides and cetearyl alcohols such as those commercially available as Montanov 68™ from Seppic and Emulgade PL68/50™ available from Henkel.

Other useful nonionic surfactants include the condensation products of alkylene oxides with fatty acids (i.e. alkylene oxide esters or diesters of fatty acids). Other nonionic surfactants are the condensation products of alkylene oxides with fatty alcohols (i.e. alkylene oxide ethers of fatty alcohols). Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. Still other useful nonionic surfactants include polyhydroxy fatty acid amide surfactants, which are described in more detail in WO98/04241.

Another emulsifier useful herein are fatty acid ester blends based on a mixture of sorbitan or sorbitol fatty acid ester and sucrose fatty acid ester, the fatty acid in each instance being preferably C₈-C₂₄, more preferably C₁₀-C₂₀. The preferred fatty acid ester emulsifier is a blend of sorbitan or sorbitol C₁₆-C₂₀ fatty acid ester with sucrose C₁₀-C₁₆ fatty acid ester, especially sorbitan stearate and sucrose cocoate. This is commercially available from ICI under the name Arlatone 2121™.

Preferred among the nonionic surfactants are those selected from the group consisting of cetearyl glucosides, cetearyl alcohols, PEG-100 stearate, sorbitan stearate and mixtures thereof.

Emulsions of the present invention may include a silicone containing emulsifier or surfactant. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. Other examples include alkyl-modified dimethicone copolyols, i.e., compounds that contain C₂-C₃₀ pendant side chains. Still other useful dimethicone copolyols include materials having various cationic, anionic, amphoteric, and zwitterionic pendant moieties.

The composition of the present invention comprises water. Preferably, water comprises from 30% to 85% by weight of the composition, more preferably 50% to 75% by weight of the composition.

The compositions of the present invention can further comprise optional ingredients. Optional ingredients are well known in the art, and can be added without reacting with and altering the chemistry therein. Optional ingredients include additional actives, neutralizing agents, sunscreening agents and mixtures thereof.

The cosmetic compositions of the present invention can be formulated in a form suitable for topical application. Non-limiting examples of such forms include self-tanning compositions, topical compositions, sprays, aerosol sprays, spray gels, lotions, creams and mousses. Preferably, the cosmetic compositions of the present invention are in the form of a self-tanning composition or a lotion.

Alternatively, the cosmetic compositions of the present invention may be used in combination with a substrate to form a skin care kit. The skin care kits of the present invention comprise the cosmetic compositions herein, and a substrate. Non-limiting examples of substrates useful herein include wet wipes, towlettes, foam sponges, roller ball delivery systems and the like. When used in this form, the substrate may be impregnated, coated, or soaked with the compositions herein. Alternatively, the compositions herein may be applied to the substrate by the consumer immediately prior to application to the skin. The skin care kits herein are desirable to prevent the accidental application of the cosmetic composition to areas of the body where it is not required or desired.

The compositions of the present invention are useful for regulating skin condition and enhancing skin tone. Such regulation of skin condition can include cosmetic prophylactic and therapeutic regulation. It may also include providing a more noticeable improvement, both tactile and visual, in the appearance and feel of the skin of a mammal. For example, such regulating methods are directed to making the skin feel softer, reducing the appearance of fine lines and wrinkles, and improving skin health. Also, such methods provide an enhancement of skin tone. The compositions of the present invention enhance skin tone by imparting a natural-looking tan to the skin of a consumer following topical application, without the appearance of an orange colour. This is believed to be due to the improved stabilisation of the self-tanning agent in the compositions, and increased delivery of the self-tanning agent in its active form.

Regulating skin condition involves topically applying to the skin a safe and effective amount of a composition of the present invention. In a preferred embodiment, the composition is chronically applied to the skin. By "chronic topical application" is meant continued topical application of the composition over an extended period during the subject's lifetime, preferably for a period of at least about one week, more preferably for a period of at least about one month, even more preferably for at least about three months, even more preferably for at least about six months, and more preferably still for at least about one year. While benefits are obtainable after various maximum periods of use (e.g., five, ten or twenty years), it is preferred that chronic application continue throughout the subject's lifetime. Typically applications would be on the order of about once per day over such extended periods, however application rates can vary from about once per week up to about three times per day or more.

A wide range of quantities of the compositions of the present invention can be employed to provide a skin appearance and/or feel benefit. Quantities of the present compositions, which are typically applied per application, are, in mg composition/cm² skin, from 0.1 mg/cm² to 30 mg/cm². A particularly useful application amount is 0.5 mg/cm² to 20 mg/cm².

Regulating skin condition is preferably practiced by applying a composition which is intended to be left on the skin for some aesthetic, cosmetic, prophylactic, therapeutic or other benefit (i.e., a "leave-on" composition). After applying the composition to the skin, it is preferably left on the skin for a period of at least 15 minutes, more preferably at least 30 minutes, even more preferably at least 1 hour, still more preferably for at least several hours, e.g., up to 12 hours.

Another approach to ensure a continuous exposure of the skin to at least a minimum level of the skin care active is to apply the compound by use of a patch applied, e.g., to the face. The patch can be occlusive, semi-occlusive or non-occlusive and can be adhesive or non-adhesive. The composition can be contained within the patch or be applied to the skin prior to application of the patch. The patch can also include additional actives such as chemical initiators for exothermic reactions such as those described in U.S. Patents numbered 5,821,250, 5,981,547, and 5,972,957. The patch is preferably left on the skin for a period of at least 5 minutes, more preferably at least 15 minutes, more preferably still at least 30 minutes, even more preferably at least 1 hour, still more preferably at night as a form of night therapy.

The compositions of the present invention may be manufactured according to a certain method. The method for manufacturing the compositions herein comprises the steps of forming a premix comprising the branched and/or cross-linked anionic polyelectrolyte polymer at a temperature of from 50°C to 70°C. Subsequently, said premix is cooled to a temperature of less than 40°C and the self-tanning agent is added. It is desirable to form the compositions of the present invention in this way to maintain the stability of the self-tanning agent during manufacture and subsequent storage. Without wishing to be bound by theory, it is believed that adding the self-tanning agent to a premix already comprising the branched and/or cross-linked anionic polyelectrolyte polymer at a temperature of less than 40°C inhibits the potential for the self-tanning agent to react with the polymer and other ingredients therein, thereby enhancing the amount of active self-tanning agent present in the composition.

### EXAMPLES I - V

| **INGREDIENTS** | **Example 1** | **Example 2** | **Example 3** | **Example 4** |
|---|---|---|---|---|
| | **(w/w%)** | **(w/w%)** | **(w/w%)** | **(w/w%)** |
| DEIONISED WATER | QS | QS | QS | QS |
| GLYCERINE | 7.0 | 10.0 | 10.0 | 10.0 |
| SK INFLUX ¹ | ----- | ----- | ----- | 1.0 |
| TOCOPHEROL NICOTINATE ² | ----- | 3.0 | ----- | ----- |
| VITAMIN E ACETATE | 0.25 | ----- | 0.5 | ----- |
| ISOHEXADECANE | 3.0 | 3.0 | 3.0 | 3.0 |
| ISOPROPYL ISOSTEARATE | 1.50 | 1.50 | 1.50 | 1.50 |
| COCONUT OIL FRACTIONATED | 0.2 | 0.2 | 0.2 | 0.2 |
| PETROLATUM | 2.0 | 2.0 | 2.0 | 2.0 |
| SIMUGEL NS ³ | 1.0 | 1.5 | 1.5 | 1.5 |
| XANTHAN GUM | 0.1 | 0.1 | 0.1 | 0.2 |
| STEARYL ALCOHOL | 0.6 | 0.6 | 0.6 | 0.6 |
| CETYL ALCOHOL | 0.5 | 0.5 | 0.5 | 0.5 |
| BEHENYL ALCOHOL | ----- | 0.4 | 0.4 | 0.4 |
| PEG-100 STEARATE | 0.1 | 0.1 | 0.1 | 0.1 |
| EMULGADE ⁴ | 0.2 | 0.2 | 0.2 | 0.2 |
| NYLONPOLY WL10 ⁵ | ----- | 1.0 | ----- | 1.0 |
| DRY FLO PLUS ⁶ | 0.5 | 1.0 | 0.5 | 1.0 |
| MICROTHENE ⁷ | 0.5 | ----- | 0.5 | ----- |
| DHA ⁸ | 1.0 | 1.5 | 2.5 | 1.5 |
| ERYTHRULOSE ⁹ | ----- | ----- | ----- | 0.5 |
| ETHYL PARABEN | 0.15 | 0.15 | 0.15 | 0.15 |
| PROPYL PARABEN | 0.07 | 0.07 | 0.07 | 0.07 |
| DISODIUM EDTA | 0.1 | 0.1 | 0.1 | 0.1 |
| BENZYL ALCOHOL | 0.25 | 0.25 | 0.25 | 0.25 |
| DC 1503 | 1.0 | 1.0 | 1.0 | 1.0 |
| PERFUME | 0.2 | 0.2 | 0.2 | 0.2 |

| | | | | |
|---|---|---|---|---|
| 1. SK Influx: Supplied by Goldschmidt AG, Goldschmidtstrasse 100, D-45127 Essen, Germany. 2. Tocopherol Nicotinate: Supplied by Ennagram UK Ltd, Edelman House, 1238 High Road, Whetstone, London. 3. Simugel NS: Supplied by Seppic, 75 Quai D'Orsay, Paris 4. Emulgade :Supplied by Cognis Deutschland GmbH, Paul-Thomas Strasse 56, D-40551 Dusseldorf, Germany. 5. Nylonpoly WL10: Supplier Optima Chemicals, Unit 17, Chiltern Business Village, Arundel Road, Uxbridge, Middlesex, UB8 2SN 6. Dry Flo : Supplied by National Starch Chemical Company, 10, Finderne Avenue, Bridgewater, NJ 08807, USA 7. Microthene: Supplied by Equistar Chemicals, 1221 McKinney Street, Suite 700, Houston, TX 77252-2583 8. DHA: Supplied by Merck GmBH, Frankfurter Strasse 250, 64293 Darmstadt, Germany. 9. Erythrulose: Supplied by Pentapharm, Engelgasse 109, 4002 Basel, Switzerland. | | | | |

The compositions are made as follows:
A water phase is prepared by admixing all water soluble ingredients (including xanthan gum), except DHA and Erythrulose, in water and heating to 80°C. A second premix is prepared by admixing of the oil soluble ingredients except the silicone oil (DC 1503) and heating also to 80°C. The oil phase is added to the water phase and sheared to form an emulsion.

The emulsion is cooled to 60°C and the polymeric thickener (Simugel NS) is then added.. At 45-50°C the benzyl alcohol and DC 1503, and particles are added and the resulting product is sheared to ensure particle dispersion, de-agglomeration and homogeneity. The composition can then be cooled to below 40°C and DHA, Erythrulose and perfume can be added. The product can then be prepared for packaging.

## Claims

1. Use of a cosmetic composition for imparting skin colouration and avoiding orange discolouration, uneven uptake of colour or a blotchy or a patchy result, said composition comprising:
a) a self-tanning agent conforming to the general formula; 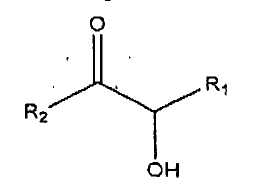 wherein R₁ is H, CH₂OH, CHOHCH₂OH, CH(OH)CH(=O), CH(OCH₃)CH(=O), CH(NH₂)CH(=O), or CH(NH-Phenyl)CH(=O); and R₂ is H or CH₂OH; and
b) a cross-linked anionic polyelectrolyte polymer, said anionic polyelectrolyte polymer comprising;
i) from 58.7% to 89.9999% by weight of the polyelectrolyte polymer of a monomer comprising a strongly acidic function;
ii) from 9.9999% to 40% by weight of the polyelectrolyte polymer of at least one hydroxy alkyl acrylate neutral monomer; and
iii) from 0.0002% to 1.3% by weight of the polyelectrolyte polymer of a diethylenic or polyethylenic cross-linking agent;
c) from 5 to 13% by weight of the composition of a polyhydric alcohol humectant wherein said humectant is glycerin
wherein said composition has a pH of from 3.5 to 4.5; and wherein the composition is in the form of an oil-in-water emulsion with a level of oil phase of from 1% to 60%.

2. Use according to claim 1 wherein the strongly acidic function of said monomer of part i) comprises a partially or totally salified sulphonic acid function, a partially or totally salified phosphonic acid function, or mixtures thereof.

3. Use according to claim 1 or claim 2 wherein the neutral monomer comprises 2-hydroxyethylacrylate, 2,3-dihydroxypropyl acrylate, 2-hydroxy-ethyl methacrylate and 2,3-dihydroxypropyl methacrylate, or an ethoxylated derivative having a molecular weight of from 400 to 1000 or mixtures thereof, preferably 2-hydroxy ethyl acrylate.

4. Use according to any one of claims 1 to 3 wherein the cross-linking agent comprises ethylene glycol dimethacrylate, sodium diallyloxyacetate, ethylene glycol diacrylate, diallylurea, trimethylolpropane triacrylate, methylene bisacrylamide or mixtures thereof, preferably methylene bisacrylamide.

5. Use according to any one of the preceding claims wherein the branched and/or cross-linked anionic polyelectrolyte polymer comprises:
i) from 58.7% to 89.9999% by weight of the polyelectrolyte polymer of the partially and/or totally salified alkali metal salt or ammonium salt of 2-methyl-2-[(1-oxo-2-propenyl)-1-propanesulphonic acid;
ii) from 9.9999% to 40% by weight of the polyelectrolyte polymer of 2-hydroxyethylacrylatc; and
iii) from 0.0002% to 1.3% by weight of the polyelectrolyte polymer of methylene bisacrylamide.

6. Use according to any one of the preceding claims wherein the branched and/or cross-linked anionic polyelectrolyte polymer is a reverse latex polymer,

7. Use according to any one of the preceding claims comprising from 0.1% to 2% of the cross-linked anionic polyelectrolyte polymer.

8. Use according to any one of the preceding claims comprising from 0.1% to 10% of the self-tann ing agent, preferably less than 5%.

9. Use according to any one of the preceding claims wherein the self-tanning agent comprises dihydroxyacetone, erythrulose, or mixtures thereof.

10. Use according to any one of the preceding claims wherein the pH of the composition is from 3.7 to 4.2.

11. Use according to any one of the preceding claims comprising from 8% to 13% by weight of the composition of said polyhydric alcohol humectant.

12. Use according to any one of the preceding claims further comprising a skin care active selected from the group consisting of betaine and its derivatives, tocopherol esters, skin lipids, or mixtures thereof.

13. A method of regulating skin condition comprising topically applying the composition according to any of the preceding claims to the skin.

14. A skin care kit comprising:
a) the composition according to any one of claims 1 to 12, and
b) a substrate.

15. A method of manufacturing the composition according to claim 1 comprising the steps of forming a premix comprising said cross-linked anionic polyelectrolyte polymer at a temperature of from 50°C to 70°C, cooling said premix to a temperature of less than 40°C and adding said self-tanning agent.

## Patentansprüche

1. Verwendung einer kosmetischen Zusammensetzung, die der Haut Farbe verleiht und die eine orange Missfärbung, eine ungleichmäßige Farbaufnahme oder ein fleckiges oder ungleichmäßiges Ergebnis vermeidet, wobei die Zusammensetzung umfasst:
a) ein Selbstbräunungsmittel, das folgender allgemeiner Formel entspricht: 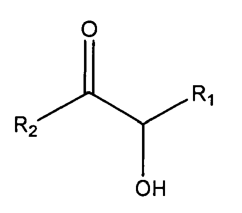 wobei R₁ für H, CH₂OH, CHOHCH₂OH, CH(OH)CH(=O), CH(OCH₃)CH(=O), CH(NH₂)CH(=O), oder CH(NH-Phenyl)CH(=O) steht, und R₂ für H oder CH₂OH steht; und
b) ein vernetztes anionisches Polyelektrolytpolymer, wobei das anionische Polyelektrolyt-Polymer umfasst;
i) zu 58,7 % bis 89,9999 %, bezogen auf das Gewicht des Polyelektrolytpolymers, ein Monomer, das eine stark saure Funktion umfasst;
ii) zu 9,9999 % bis 40 %, bezogen auf das Gewicht des Polyelektrolyt-Polymers, mindestens ein neutrales Hydroxyalkylacrylat-Monomer; und
iii) zu 0,0002 % bis 1,3 %, bezogen auf das Gewicht des Polyelektrolyt-Polymers, ein Diethylen- oder Polyethylen-Vernetzungsmittel;
c) zu 5 bis 13 Gew.-% der Zusammensetzung ein mehrwertiges Alkohol-Befeuchtungsmittel, wobei es sich bei dem Befeuchtungsmittel um Glycerin handelt;
wobei die Zusammensetzung einen pH-Wert von 3,5 bis 4,5 aufweist; und wobei die Zusammensetzung in Form einer Öl-in-Wasser-Emulsion mit einem Anteil der Ölphase von 1 % bis 60 % vorliegt.

2. Verwendung nach Anspruch 1, wobei die stark saure Funktion des Monomers von Teil i) eine teilweise oder vollständig versalzte Sulphonsäurefunktion, eine teilweise oder vollständig versalzte Phosphonsäurefunktion oder Mischungen davon umfasst.

3. Verwendung nach Anspruch 1 oder Anspruch 2, wobei das neutrale Monomer 2-Hydroxyethylacrylat, 2,3-Dihydroxypropylacrylat, 2-Hydroxyethylmethacrylat und 2,3-Dihydroxypropylmethacrylat oder ein ethoxyliertes Derivat mit einem Molekulargewicht von 400 bis 1000 oder Mischungen davon, vorzugsweise 2-Hydroxyethylacrylat, umfasst.

4. Verwendung nach einem der Ansprüche 1 bis 3, wobei das Vernetzungsmittel Ethylenglycoldimethacrylat, Natriumdiallyloxyacetat, Ethylenglycoldiacrylat, Diallylurea, Trimethylolpropantriacrylat, Methylenbisacrylamid oder Mischungen davon, vorzugsweise Methylenbisacrylamid, umfasst.

5. Verwendung nach einem der vorangehenden Ansprüche, wobei das verzweigte und/oder vernetzte anionische Polyelektrolyt-Polymer umfasst:
i) zu 58,7 % bis 89,9999 %, bezogen auf das Gewicht des Polyelektrolyt-Polymers, das teilweise und/oder vollständig versalzte Alkalimetallsalz oder Ammoniumsalz von 2-Methyl-2-[(1-oxo-2-propenyl)-1-propansulphonsäure;
ii) zu 9,9999 % bis 40 %, bezogen auf das Gewicht des Polyelektrolyt-Polymers, 2-Hydroxyethylacrylat; und
iii) zu 0,0002 % bis 1,3 %, bezogen auf das Gewicht des Polyelektrolyt-Polymers, Methylenbisacrylamid.

6. Verwendung nach einem der vorangehenden Ansprüche, wobei das verzweigte und/oder vernetzte anionische Polyelektrolyt-Polymer ein Umkehr-Latexpolymer ist.

7. Verwendung nach einem der vorangehenden Ansprüche, 0,1 % bis 2 % des vernetzten anionischen Polyelektrolyt-Polymers umfassend.

8. Verwendung nach einem der vorangehenden Ansprüche, 0,1 % bis 10 %, bevorzugt weniger als 5 % Selbstbräunungsmittel umfassend.

9. Verwendung nach einem der vorangehenden Ansprüche, wobei das Selbstbräunungsmittel Dihydroxyaceton, Erythrulose oder Mischungen davon umfasst.

10. Verwendung nach einem der vorangehenden Ansprüche, wobei der pH-Wert der Zusammensetzung 3,7 bis 4,2 beträgt.

11. Verwendung nach einem der vorangehenden Ansprüche, zu 8 % bis 13 %, bezogen auf das Gewicht der Zusammensetzung, das mehrwertige Alkoholbefeuchtungsmittel umfassend.

12. Verwendung nach einem der vorangehenden Ansprüche, ferner einen Hautpflege-Wirkstoff umfassend, der ausgewählt ist aus der Gruppe bestehend aus Betain und seinen Derivaten, Tocopherolestern, Hautlipiden oder Mischungen davon.

13. Verfahren zur Regulierung der Hautbeschaffenheit, umfassend das topische Auftragen der Zusammensetzung auf die Haut nach einem der vorangehenden Ansprüche.

14. Hautpflege-Kit, umfassend:
a) die Zusammensetzung nach einem der Ansprüche 1 bis 12, und
b) ein Substrat.

15. Verfahren zur Herstellung der Zusammensetzung nach Anspruch 1, umfassend die Schritte des Ausbildens einer Vormischung, die das vernetzte anionische Polyelektrolyt-Polymer umfasst, bei einer Temperatur von 50 °C bis 70 °C, des Abkühlens der Vormischung auf eine Temperatur unter 40 °C und des Zugebens des Selbstbräunungsmittels.

## Revendications

1. Utilisation d'une composition cosmétique apportant une coloration de la peau et évitant une décoloration orange, une absorption irrégulière ou un résultat taché ou marbré, ladite composition comprenant :
a) un agent auto-bronzant répondant à la formule générale ; 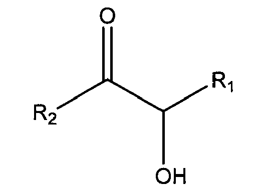 dans laquelle R₁ est H, CH₂OH, CHOHCH₂OH, CH(OH)CH(=O), CH(OCH₃)CH(=O), CH(NH₂)CH(=O), ou CH(NH-phényl)CH(=O) ; et R₂ est H ou CH₂OH ; et
b) un polymère polyélectrolyte anionique réticulé, ledit polymère polyélectrolyte anionique comprenant ;
i) de 58,7 % à 89,9999 % en poids du polymère polyélectrolyte d'un monomère comprenant une fonction fortement acide ;
ii) de 9,9999 % à 40 % en poids du polymère polyélectrolyte d'au moins un monomère neutre acrylate d'hydroxyalkyle ; et
iii) de 0,0002 % à 1,3 % en poids du polymère polyélectrolyte d'un agent de réticulation diéthylénique ou polyéthylénique ;
c) de 5 à 13 % en poids de la composition d'un humectant alcool polyhydrique dans laquelle ledit humectant est de la glycérine ;
dans laquelle ladite composition a un pH de 3,5 à 4,5 ; et dans laquelle la composition est sous la forme d'une émulsion huile-dans-eau avec un taux de phase huileuse allant de 1 % à 60 %

2. Utilisation selon la revendication 1, dans laquelle la fonction fortement acide dudit monomère de la partie i) comprend une fonction acide sulfonique partiellement ou totalement salifiée, une fonction acide phosphonique partiellement ou totalement salifiée, ou leurs mélanges.

3. Utilisation selon la revendication 1 ou la revendication 2, dans laquelle le monomère neutre comprend du 2-hydroxyéthylacrylate, du 2,3-dihydroxypropyl acrylate, du 2-hydroxy-éthyl méthacrylate et du 2,3-dihydroxypropyl méthacrylate, ou un dérivé éthoxylé ayant une masse moléculaire allant de 400 à 1000 ou leurs mélanges, de préférence du 2-hydroxy éthyl acrylate.

4. Utilisation selon l'une quelconque des revendications 1 à 3, dans laquelle l'agent de réticulation comprend du diméthacrylate d'éthylène glycol, du diallyloxyacétate de sodium, du diacrylate d'éthylène glycol, de la diallylurée, du triacrylate de triémthylolpropane, du méthylène bisacrylamide ou leurs mélanges, de préférence du méthylène bisacrylamide.

5. Utilisation selon l'une quelconque des revendications précédentes, dans laquelle le polymère polyélectrolyte anionique ramifié et/ou réticulé comprend :
i) de 58,7 % à 89,9999 % en poids du polymère polyélectrolyte du sel de métal alcalin ou du sel d'ammonium partiellement et/ou totalement salifié d'acide 2-méthyl-2-[(1-oxo-2-propényl)-1-propanesulfonique ;
ii) de 9,9999 % à 40 % en poids du polymère polyélectrolyte de 2-hydroxyéthylacrylate ; et
iii) de 0,0002 % à 1,3 % en poids du polymère polyélectrolyte de méthylène bisacrylamide.

6. Utilisation selon l'une quelconque des revendications précédentes, dans laquelle le polymère polyélectrolyte anionique ramifié et/ou réticulé est un polymère de latex inverse.

7. Utilisation selon l'une quelconque des revendications précédentes, comprenant de 0,1 % à 2 % du polymère polyélectrolyte anionique réticulé.

8. Utilisation selon l'une quelconque des revendications précédentes, comprenant de 0,1 % à 10 % de l'agent auto-bronzant, de préférence moins de 5 %.

9. Utilisation selon l'une quelconque des revendications précédentes, dans laquelle l'agent auto-bronzant comprend du dihydroxyacétone, de l'érythrulose, ou leurs mélanges.

10. Utilisation selon l'une quelconque des revendications précédentes, dans laquelle le pH de la composition va de 3,7 à 4,2.

11. Utilisation selon l'une quelconque des revendications précédentes, comprenant de 8 % à 13 % en poids de la composition dudit humectant alcool polyhydrique.

12. Utilisation selon l'une quelconque des revendications précédentes, comprenant en outre un principe actif pour le soin de la peau choisi dans le groupe constitué de la bétaïne et ses dérivés, des esters de tocophérol, des lipides cutanés, ou leurs mélanges.

13. Procédé de régulation de l'état de la peau, comprenant l'application locale de la composition selon l'une quelconque des revendications précédentes sur la peau.

14. Trousse de soin de la peau comprenant :
a) la composition selon l'une quelconque des revendications 1 à 12, et
b) un substrat.

15. Procédé de fabrication de la composition selon la revendication 1, comprenant les étapes consistant à former un prémélange comprenant ledit polymère polyélectrolyte anionique réticulé à une température allant de 50 °C à 70 °C, refroidir ledit prémélange à une température de moins de 40 °C et ajouter ledit agent auto-bronzant.
